# EUROPÄISCHE PATENTSCHRIFT

(11) **EP 2 441 393 B1**
(45) Veröffentlichungstag und Bekanntmachung des Hinweises auf die Patenterteilung: **06.03.2013**
(21) Anmeldenummer: 10013650.6
(22) Anmeldetag: 14.10.2010
(51) Int. Cl.: A61B 17/00, H01H 9/04, H01H 13/70, H01H 13/06

(54) **Chirurgisches Instrument**
Surgical instrument
Instrument chirurgical

(43) Veröffentlichungstag der Anmeldung: 18.04.2012
(73) Patentinhaber: Eberle GmbH & Co. KG, 75449 Wurmberg (DE)
(72) Erfinder: Löffler, Heiko, 75449 Wurmberg (DE)
(74) Vertreter: Schaeberle, Steffen

(56) Entgegenhaltungen:
- EP-A2- 0 965 568
- WO-A2-2004/026150
- US-A1- 2007 081 348
- US-B1- 6 402 748

## Beschreibung

Die vorliegende Erfindung betrifft ein chirurgisches Instrument mit einem Handgriff und einem am Handgriff angeordneten Bedienungselement.

Chirurgische Instrumente mit Handgriffen sind aus dem Stand der Technik in unterschiedlichen Ausgestaltungen bekannt. Die Handgriffe sind häufig als hohle Griffe ausgebildet, wobei eine Antriebswelle oder Ähnliches. durch den hohlen Handgriff geführt ist. Dabei können am Handgriff direkt Bedienelemente für ein Einschalten bzw. Ausschalten des chirurgischen Instruments angeordnet sein. Da der Handgriff während einer Operation mit Körperflüssigkeiten oder anderen Flüssigkeiten in Kontakt kommen kann, muss ein gewisser Schutz der Bedienelemente vorgesehen werden. Besonders kritisch für die Instrumente sind jedoch die nach einer Operation notwendigen Reinigungsarbeiten, insbesondere bei einer Wiederaufbereitung mittels Dampfsterilisation. Hierbei wurde vorgeschlagen, die Bedienelemente mittels einer Folie abzudecken. Hierbei hat sich jedoch herausgestellt, dass insbesondere die Montage der Folie relativ zeitaufwändig und damit kostenintensiv ist. Ein weiterer Nachteil der bekannten Lösung besteht darin, dass nach einer Vielzahl von Benutzungen die Folie einreißen kann. Derartige Beschädigungen der Folie führen jedoch zu unerwünschten Undichtigkeiten. Ein solches chirurgisches Instrument ist z.B. aus dem US 6 402 748 B1 bekannt.

Es ist daher Aufgabe der vorliegenden Erfindung, ein chirurgisches Instrument mit Handgriff und Bedienelementen bereitzustellen, welches bei einfachem Aufbau und einfacher, kostengünstiger Herstellbarkeit eine dauerhafte Gewährleistung einer Funktion sicherstellen kann, auch wenn das chirurgische Instrument mit Körperflüssigkeiten oder anderen Flüssigkeiten in Berührung kommt.

Diese Aufgabe wird durch ein chirurgisches Instrument mit den Merkmalen des Anspruches 1 gelöst. Die Unteransprüche zeigen bevorzugte Weiterbildungen der Erfindung.

Das erfindungsgemäße chirurgische Instrument weist somit den Vorteil auf, dass es eine sichere Abdichtung gegenüber möglicherweise eindringenden Flüssigkeiten aufweist, wobei erfindungsgemäß eine lange Lebensdauer sichergestellt ist. Eine Schaltplatine übernimmt dabei eine fluiddichte Trennung eines inneren Bereichs des Gehäuses von einer Umgebung. Somit weist die Schaltplatine eine Doppelfunktion eines Schaltvorganges und einer fluiddichten Trennung eines inneren Gehäusebereichs von einer Außenseite auf. Hierbei ist kein separates Trennelement, wie z.B. eine Folie oder Ähnliches, notwendig. Erfindungsgemäß ist ein Schalter in einer Durchgangsöffnung eines Handgriffs angeordnet, wobei der Schalter ein Betätigungselement, die Schaltplatine sowie ein federndes Kontaktelement, insbesondere eine aus leitendem Material hergestellte Federscheibe oder ein mit elektrisch leitendem Material beschichtetes Federelement, aufweist. Eine Abdichtung erfolgt dabei mittels der Schaltplatine und eines Dichtelements, welches an einer Unterseite der Schaltplatine angeordnet ist. Das Dichtelement dichtet dabei gegen das Gehäuse des Handgriffs ab. Das Dichtelement ist umlaufend geschlossen, so dass an der Schaltplatine in nach innen gerichteter Richtung ausgehend vom Dichtelement empfindliche Bauteile oder Kontakte angeordnet werden können, welche vor dem Eindringen von Feuchtigkeit geschützt sind. Somit ist erfindungsgemäß eine Unterseite der Schaltplatine vor Feuchtigkeit geschützt.

Vorzugsweise ist im Gehäuse benachbart zu der Durchgangsöffnung, in welcher der Schalter angeordnet ist, eine Nut vorgesehen, in welcher das Dichtelement angeordnet ist. Hierdurch kann einerseits die Montage erleichtert werden und andererseits kann das Dichtelement sich hervorragend an die Wände der Nut anlegen und dort abdichten.

Gemäß einer weiteren bevorzugten Ausgestaltung der Erfindung umfasst der Schalter eine Führungsscheibe zur Führung des Betätigungselements. Hierdurch wird eine sichere Funktion des Schalters ermöglicht. Das Betätigungselement weist vorzugsweise einen Führungszapfen auf, welcher in einer in der Führungsscheibe ausgebildeten Ausnehmung geführt ist.

Weiterhin bevorzugt umfasst der Schalter eine Abdeckung, welche am Handgriff fixiert ist, wobei die Abdeckung wenigstens eine Öffnung zur Aufnahme des Betätigungselements umfasst.

Weiter bevorzugt umfasst das Betätigungselement wenigstens zwei Nasen, vorzugsweise drei Nasen, welche hinter eine in der Abdeckung gebildete Hinterschneidung greifen. Hierdurch wird vermieden, dass das Betätigungselement aus der Abdeckung herausfallen kann.

Gemäß einer weiteren bevorzugten Ausgestaltung der Erfindung liegt die Führungsscheibe auf der Schaltplatine auf. Dadurch kann über die Führungsscheibe eine Vorspannung auf die Schaltplatine ausgeübt werden, wobei die Schaltplatine ihrerseits die Vorspannkraft auf das Dichtelement überträgt. Hierdurch kann eine besonders gute und sichere Abdichtung erreicht werden.

Weiter bevorzugt umfasst die Federscheibe eine Vielzahl von Kontaktfüßen an einem Außenumfang, welcher mit einem Kontakt an der Schaltplatine in Kontakt stehen. Hierdurch kann beispielsweise beim Drücken des Betätigungselements ein mittlerer Bereich der Federscheibe auf die Schaltplatine gedrückt werden und so ein elektrischer Kontakt über die Federscheibe hergestellt werden.

Das umlaufende Dichtelement ist vorzugsweise als O-Ring ausgebildet. Hierdurch kann ein besonders kostengünstiger Aufbau erreicht werden.

In der nachfolgenden Zeichnung wird ein erfindungsgemäßes chirurgisches Instrument unter Bezugnahme auf die Figur im Detail beschrieben. In der Zeichnung ist:
- Fig. 1: eine schematische Draufsicht eines chirurgischen Instruments mit einem handbetätigten Schalter gemäß einem Ausführungsbeispiel der Erfindung,
- Fig. 2: eine schematische, perspektivische Explosionsdarstellung des chirurgischen Instruments mit handbetätigtem Schalter, und
- Fig. 3: eine schematische Schnittansicht des handbetätigten Schalters gemäß der Erfindung.

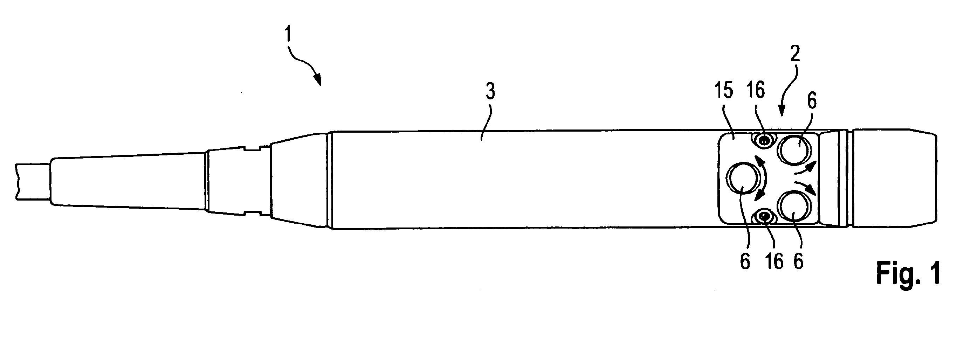

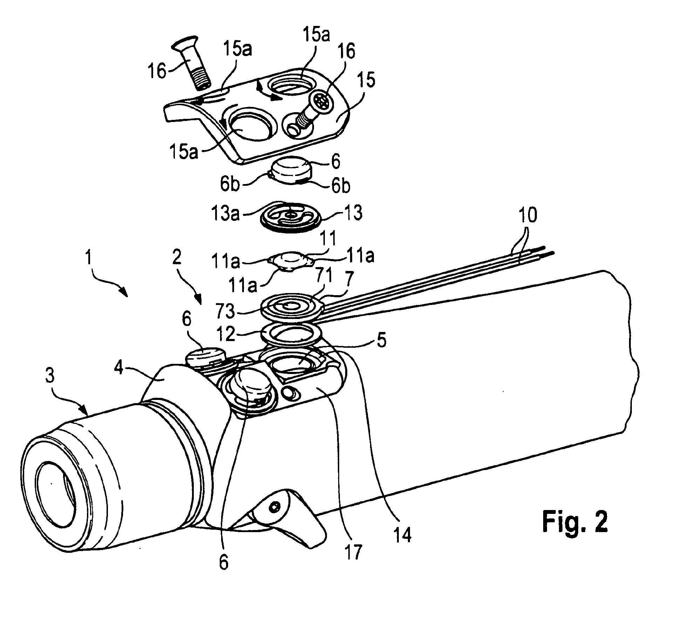

Nachfolgend wird unter Bezugnahme auf die Fig. 1 bis 3 ein chirurgisches Instrument 1 mit einem Handgriff 3 und einem handbetätigten Schalter 2 im Detail beschrieben. Wie insbesondere aus Fig. 2 ersichtlich ist, ist der Handgriff 3 ein im Wesentlichen zylinderförmiger, hohler Handgriff, durch welchen eine Welle oder Ähnliches geführt ist. Der Handgriff 3 umfasst ein Gehäuse 4, in welchem eine Durchgangsöffnung 5 vorgesehen ist. Der handbetätigte Schalter 2 ist dabei in der Durchgangsöffnung 5 angeordnet.

Der handbetätigte Schalter 2 umfasst ein Betätigungselement 6 in Form eines Druckknopfes, wobei das Betätigungselement 6 einen zentralen Führungszapfen 6a sowie drei radial nach außen vorstehende Nasen 6b umfasst. Der handbetätigte Schalter 2 umfasst ferner eine Schaltplatine 7 mit einer Oberseite 8 und einer Unterseite 9, als federndes Kontaktelement eine elektrisch leitfähige Federscheibe 11, eine Führungsscheibe 13 und ein Dichtelement 12. Wie insbesondere aus Fig. 1 ersichtlich ist, umfasst der handbetätigte Schalter dieses Ausführungsbeispiels insgesamt drei einzelne Schaltvorrichtungen, beispielsweise eine Schaltvorrichtung für einen Vorlauf, eine Schaltvorrichtung für einen Rücklauf und eine Schaltvorrichtung für eine oszillierende Bewegung des chirurgischen Instruments. Wie aus den Fig. 1 und 2 ersichtlich ist, sind die drei Schaltvorrichtungen dabei unmittelbar benachbart zueinander angeordnet, wobei im Gehäuse 4 des Handgriffs 3 eine entsprechende Vertiefung 17 vorgesehen ist (vgl. Fig. 2).

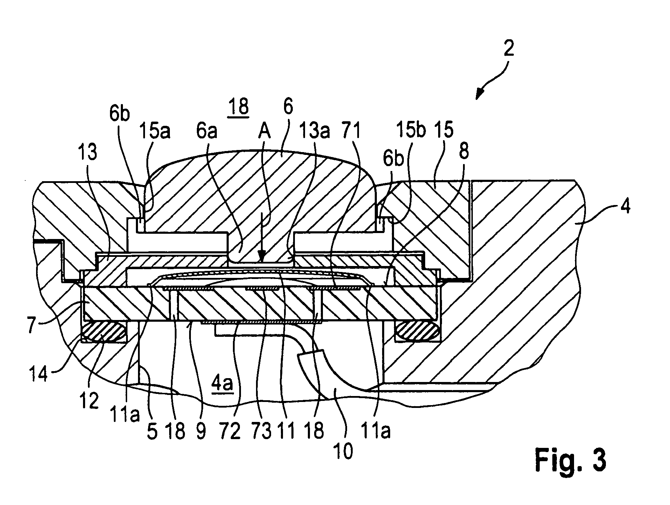

Wie insbesondere aus Fig. 3 ersichtlich ist, ist das Betätigungselement 6 in einer Öffnung 15a einer Abdeckung 15 angeordnet. Die Abdeckung 15 umfasst dabei eine Hinterschneidung 15b, hinter welche die Nasen 6b des Betätigungselements 6 greifen. Der Führungszapfen 6a des Betätigungselements 6 ist in einer Führungsöffnung 13a der Führungsscheibe 13 angeordnet. Die Führungsscheibe 13 liegt unmittelbar auf der Oberseite 8 der Schaltplatine 7 auf. Das Dichtelement 12 in diesem Ausführungsbeispiel ist ein O-Ring, welcher an der Unterseite 9 der Schaltplatine 7 angeordnet ist. An der Oberseite 8 der Schaltplatine 7 ist ein erster Kontakt 71 und an der Unterseite 9 ein zweiter Kontakt 72 vorgesehen. Wie aus Fig. 2 ersichtlich ist, ist die Schaltplatine 7 des Ausführungsbeispiels ringförmig. An der Unterseite 9 der Schaltplatine 7 sind zwei Leitungen 10 in bekannter Weise am zweiten Kontakt 72 befestigt. Die Leitungen 10 sind durch den hohlen Handgriff zu einer Motorsteuerung des chirurgischen Instruments geführt.

Wie insbesondere aus Fig. 3 ersichtlich ist, ist das Dichtelement 12 in einer umlaufenden, U-förmigen Nut 14 angeordnet. Dabei ist die Schaltplatine 7 derart angeordnet, dass die Schaltplatine 7 selbst eine fluiddichte Trennung von einem inneren Bereich 4a des Gehäuses zu einem Umgebungsbereich 18 bereitstellt. Somit weist die Schaltplatine 4 neben der Bereitstellung einer Schaltfunktion auch eine Trennfunktion zwischen dem inneren Bereich 4a und dem Umgebungsbereich 18 auf. Hierdurch kann erfindungsgemäß die Schaltplatine 7 als Trennelement verwendet werden, so dass kein zusätzliches Bauteil, wie z.B. eine Folie oder Ähnliches, notwendig ist. Somit können die Teilezahl und die Herstellungskosten reduziert werden. Die plattenförmige Abdeckung 15 dieses Ausführungsbeispiels weist drei Öffnungen 15a auf, um insgesamt drei Schaltvorrichtungen aufzunehmen. Wie aus Fig. 2 ersichtlich ist, wird die Abdeckung 15 mittels zweier Schrauben 16 in das Gehäuse 4 eingeschraubt und dort befestigt. Hierbei ist im Gehäuse 4 eine Vertiefung 17 ausgebildet, um die Abdeckung 15 vollständig aufzunehmen, so dass die Abdeckung 15 nicht an einer Außenseite des Handgriffs vorsteht.

Wie insbesondere aus Fig. 3 ersichtlich ist, drückt somit im montierten Zustand die Abdeckung 15 gegen die Führungsscheibe 13, welche auf der Oberseite 8 der Schaltplatine 7 aufliegt. Dadurch wird die Schaltplatine 7 ebenfalls radial nach innen gegen das Dichtelement 12 gedrückt.

Wenn nun ein Schalter betätigt werden soll, übt eine Bedienperson beispielsweise mit einem Finger einen radial nach innen gerichteten Druck auf das Betätigungselement 6 aus. Dieses wird, wie in Fig. 3 durch den Pfeil A angedeutet, radial nach innen gedrückt und drückt auf einen mittleren Bereich der Federscheibe 11. Wie aus Fig. 2 ersichtlich ist, weist die Federscheibe 11 vier Kontaktfüßchen 11a auf, welche an dem an der Oberseite 8 der Schaltplatine 7 gebildeten ersten Kontakt 71 aufliegen. Ein weiterer mittlerer Kontakt 73 ist an der Oberseite unter dem mittleren Bereich der Federscheibe 11 gebildet. Wenn nun das Betätigungselement 6 in Pfeilrichtung A gedrückt wird, kommt dieses mit der Federscheibe 11 in Kontakt und drückt die Mitte der Federscheibe 11 gegen den mittleren Kontakt 73. Über eine durch die Schaltplatine 7 vorgesehene Durchkontaktierung 18 ist somit eine Verbindung zwischen den Kontakten an der Oberseite und der Unterseite, an denen die Leitungen 10 fixiert sind, hergestellt. Dadurch kann das chirurgische Instrument angetrieben werden.

Dabei wird immer eine ausreichende Dichtheit über die Schaltplatine 7 und das Dichtelement 12 sichergestellt, damit keine Feuchtigkeit in den inneren Bereich 4a des Gehäuses 4 dringen kann. Es sei angemerkt, dass für die Funktion des chirurgischen Instruments es unwesentlich ist, sollte Feuchtigkeit beispielsweise bis zur Oberseite 8 der Schaltplatine 7 gelangen. Allerdings liegt die Abdeckung 15 praktisch fluiddicht auf der Führungsscheibe 13 auf und die Führungsscheibe 13 wird dadurch auf die Oberseite 8 der Schaltplatine 7 gedrückt. Ferner ist insbesondere nur ein minimales Spiel im Bereich der Führungsöffnung 13a zum Führungszapfen 6a vorhanden, so dass auch von hier im Betrieb oder bei einer Reinigung nur bedingt Feuchtigkeit eindringen kann.

Die Erfindung weist ferner den Vorteil auf, dass gegebenenfalls der komplette Schalter 2 wieder demontiert werden kann und beispielsweise bei einem Defekt eines Bauteils dieses einfach ausgetauscht werden kann. Trotzdem kann auch nach einer Wiedermontage des Schalters immer sichergestellt werden, dass keine Feuchtigkeit bis in den inneren Bereich 4a des Gehäuses 4 eindringen kann. Auch ist insbesondere die Montage des Schalters 2 deutlich vereinfacht, da lediglich die Bauteile in der Reihenfolge Dichtelement 12, Schaltplatine 7, Federscheibe 11, Führungsscheibe 13 und Schaltelement 6 übereinander angeordnet werden müssen und dann wird lediglich die Abdeckung 15 mittels Schrauben 16 am Gehäuse 4 fixiert. Hierbei kann insbesondere das Dichtelement 12 einfach in die Nut 14 eingelegt werden, so dass bei jeder Montage immer eine Dichtheit gewährleistet ist.

### Bezugszeichenliste

- 1: Chirurgisches Instrument
- 2: Schalter
- 3: Handgriff
- 4: Gehäuse
- 4a: Innerer Bereich
- 5: Durchgangsöffnung
- 6: Betätigungselement
- 6a: Führungszapfen
- 6b: Nase
- 7: Schaltplatine
- 8: Oberseite
- 9: Unterseite
- 10: Leitung
- 11: Federscheibe
- 12: Dichtelement
- 13: Führungsscheibe
- 14: Nut
- 15: Abdeckung
- 15a: Öffnung
- 15b: Hinterschneidung
- 16: Schraube
- 17: Vertiefung
- 18: Umgebungsbereich
- 71: erster Kontakt
- 72: zweiter Kontakt
- 73: mittlerer Kontakt
- A: Druckkraft

## Patentansprüche

1. Chirurgisches Instrument mit wenigstens einem handbetätigtem Schalter (2), umfassend
- einen Handgriff (3) mit einem Gehäuse (4), in welchem eine Durchgangsöffnung (5) zur Aufnahme des Schalters (2) angeordnet ist, wobei der Schalter (2) umfasst:
- ein Betätigungselement (6),
- eine Schaltplatine (7) mit einem ersten Kontakt (71) an einer Oberseite (8)
- ein federndes Kontaktelement (11), welches zwischen dem Betätigungselement (6) und der Schaltplatine (7) angeordnet ist, **dadurch gekennzeichnet, dass** der Schalter (2) umfasst einen zweiten Kontakt (72) an einer Unterseite (9) der Schatplatine (2),
- ein umlaufend ausgebildetes Dichtelement (12), welches an der Unterseite (9) der Schaltplatine (7) angeordnet ist und eine Abdichtung zwischen dem Gehäuse (4) des Handgriffs und der Schaltplatine (7) bereitstellt,
- wobei die Schaltplatine einen inneren Bereich (4a) im Gehäuse (4) von einem Umgebungsbereich (18) fluiddicht trennt.

2. Instrument nach Anspruch 1, **gekennzeichnet durch** eine im Gehäuse (4) benachbart zur Durchgangsöffnung (5) angeordnete Nut (14), in welcher das Dichtelement (12) angeordnet ist.

3. Instrument nach einem der vorhergehenden Ansprüche, ferner umfassend eine Führungsscheibe (13) zur Führung des Betätigungselements (6).

4. Instrument nach Anspruch 3, **dadurch gekennzeichnet, dass** das Betätigungselement (6) einen Führungszapfen (6a) umfasst und die Führungsscheibe (13) eine Führungsöffnung (13a) umfasst, wobei der Führungszapfen (6a) in der Führungsöffnung (13a) angeordnet ist.

5. Instrument nach einem der vorherigen Ansprüche, ferner umfassend eine Abdeckung (15), welche am Gehäuse (4) des Handgriffs (3) an der Durchgangsöffnung (5) fixiert ist, wobei die Abdeckung (15) eine Öffnung (15a) zur Aufnahme des Betätigungselements (6) umfasst.

6. Instrument nach Anspruch 5, **dadurch gekennzeichnet, dass** das Betätigungselement (6) wenigstens zwei Nasen (6b) aufweist, welche radial nach außen vorstehen und wobei die Abdeckung (15) eine Hinterschneidung (15b) umfasst, hinter welche die Nasen (6b) greifen.

7. Instrument nach einem der Ansprüche 3 bis 6, **dadurch gekennzeichnet, dass** die Führungsscheibe (13) mit der Schaltplatine (7) in Kontakt ist.

8. Instrument nach einem der vorherigen Ansprüche, **dadurch gekennzeichnet, dass** das federnde Kontaktelement (11) eine Vielzahl von Kontaktfüßen (11a) an einem Außenumfang aufweist, wobei die Kontaktfüße (11a) mit einem ersten Kontakt (71) an der Oberseite (8) der Schaltplatine in Kontakt stehen.

9. Instrument nach einem der vorherigen Ansprüche, **dadurch gekennzeichnet, dass** das Dichtelement (12) ein O-Ring ist.

## Claims

1. A surgical instrument having at least one hand-operated switch (2), comprising:
- a handle (3) having a housing (4), in which a passage opening (5) for accommodating the switch (2) is arranged, wherein the switch (2) comprises:
- an operating element (6),
- a circuit board (7) having a first contact (71) on an upper face (8),
- a resilient contact element (11) which is arranged between the operating element (6) and the circuit board (7),
- a second contact (72) on a lower face (9) of the circuit board,
**characterized in that** the switch (2) comprises:
- a circumferential sealing element (12) which is arranged on the lower face (9) of the circuit board (7) and provides a seal between the housing (4) of the handle and the circuit board (7),
- wherein the circuit board separates an inner region (4a) in the housing (4) from a surrounding region (18) in a fluid-tight manner.

2. The instrument according to claim 1, **characterized by** a groove (14) which is arranged in the housing (4) next to the passage opening (5) and in which the sealing element (12) is arranged.

3. The instrument according to any one of the preceding claims, further comprising a guide disk (13) for guiding the operating element (6).

4. The instrument according to claim 3, **characterized in that** the operating element (6) comprises a guide pin (6a) and the guide disk (13) comprises a guide opening (13a), wherein the guide pin (6a) is arranged in the guide opening (13a).

5. The instrument according to any one of the preceding claims, further comprising a cover (15) which is fixed on the housing (4) of the handle (3) on the passage opening (5), wherein the cover (15) comprises an opening (15a) for accommodating the operating element (6).

6. The instrument according to claim 5, **characterized in that** the operating element (6) comprises at least two noses (6b) projecting radially outwards, and wherein the cover (15) has an undercut (15b) which the noses (6b) engage behind.

7. The instrument according to any one of claims 3 to 6, **characterized in that** the guide disk (13) is in contact with the circuit board (7).

8. The instrument according to any one of the preceding claims, **characterized in that** the resilient contact element (11) comprises a plurality of contact feet (11 a) on an outer circumference, wherein the contact feet (11 a) are in contact with a first contact (71) on the upper face (8) of the circuit board.

9. The instrument according to any one of the preceding claims, **characterized in that** the sealing element (12) is an O-ring.

## Revendications

1. Instrument chirurgical avec au moins un commutateur (2) actionné à main, comprenant
- un manche (3) avec un boîtier (4) dans lequel est disposé une ouverture de passage (5) pour recevoir le commutateur (2), le commutateur comprenant:
- un élément d'actionnement (6)
- un circuit imprimé (7) avec un premier contact (71) sur un coté supérieur (8),
- un élément de contact (11) élastiquet qui est disposé entre l'élément d'actionnement (6) et le circuit imprimé (7),
- un deuxième contact (72) sur un dessous (9) du circuit imprimé (7),
**caractérisé en ce que** le commutateur (2) comprend:
- un élement d'étanchéité (12) formé d'une manière circulaire qui est disposé sur le dessous (9) du circuit imprimé (7) et qui met en place un étanchement entre le boîtier (4) du manche et le circuit imprimé (7),
- le circuit imprimé (7) séparant une zone intérieure (4a) dans le boîtier (4) d'une zone environnante (18) d'une manière étanche pour un fluide.

2. Instrument selon revendication 1, **caractérisé par** une rainure (14) disposée dans le boîtier (4) et avoisinant l'ouverture de passage (5), avec l'élément d'étanchéité (12) étant disposé dans la rainure.

3. Instrument selon l'une quelquonque des revendications précédentes comprenant en outre un disque de guidage (13) pour guider l'élément d'actionnement (6).

4. Instrument selon revendication 3, **caractérisé en ce que** l'élément d'actionnement (6) comprend un tenon de guidage (6a) et que le disque de guidage (13) comprend une ouverture de guidage (13a), avec le tenon de guidage (6a) étant disposé dans l'ouverture de guidage (13a).

5. Instrument selon l'une quelquonque des revendications précédentes comprenant en outre un couvercle (15) qui est attaché au boîtier (4) du manche (3) à l'ouverture de passage (5), avec le couvercle (15) comprenant une ouverture (15a) pour recevoir l'élément d'actionnement (6).

6. Instrument selon revendication 5 **caractérisé en ce que** l'élément d'actionnement (6) comprend au moins deux nez (6b) qui débordent radialement vers l'extérieure, et le couvercle (15) comprenant un dégagement (15b) derrière lequel les nez (6b) s'engrènent.

7. Instrument selon une des revendication 3 à 6 **caractérisé en ce que** le disque de guidage (13) est en contact avec le circuit imprimé (7).

8. Instrument selon l'une quelquonque des revendications précédentes **caractérisé en ce que** l'élément de contact (11) élastique comporte une multitude de patins de contact (11a) à une périphérie extérieure, les patins de contact (11 a) étant en contact avec un premier contact (71) sur le coté supérieur (8) du circuit imprimé.

9. Instrument selon l'une quelquonque des revendications précédentes **caractérisé en ce que** l'élement d'étanchéité (12) est un joint torique.
